# EUROPEAN PATENT SPECIFICATION

(11) **EP 2 717 771 B1**
(45) Date of publication and mention of the grant of the patent: **03.10.2018**
(21) Application number: 12729235.7
(22) Date of filing: 05.06.2012
(51) Int. Cl.: A61B 34/20

(54) **OPTICAL FIBER SENSING FOR DETERMINING REAL TIME CHANGES IN APPLICATOR GEOMETRY FOR INTERVENTIONAL THERAPY**
GLASFASERMESSUNG ZUR BESTIMMUNG VON ECHTZEIT-VERÄNDERUNGEN EINER APPLIKATORGEOMETRIE FÜR INTERVENTIONELLE THERAPIE
DÉTECTION DE FIBRE OPTIQUE POUR DÉTERMINER DES CHANGEMENTS EN TEMPS RÉEL DANS UNE GÉOMÉTRIE D'APPLICATEUR POUR UNE THÉRAPIE D'INTERVENTION

(30) Priority: 10.06.2011 US 201161495906 P; 03.12.2011 US 201161566619 P
(43) Date of publication of application: 16.04.2014
(73) Proprietor: Koninklijke Philips N.V., 5656 AE Eindhoven (NL)
(72) Inventor: VON BUSCH, Heinrich Johannes, NL-5656 AE Eindhoven (NL); CHAN, Raymond, NL-5656 AE Eindhoven (NL); 'T HOOFT, Gert Wim, NL-5656 AE Eindhoven (NL); AARNINK, Reinardus Gerhardus, NL-5656 AE Eindhoven (NL); DESJARDINS, Adrien Emmanuel, NL-5656 AE Eindhoven (NL)
(74) Representative: Steffen, Thomas
(86) International application number: PCT/IB2012/052816
(87) International publication number: WO 2012/168855

(56) References cited:
- US-A1- 2001 021 843
- US-A1- 2008 071 143
- US-A1- 2009 137 952

## Description

This disclosure relates to medical devices, and more particularly to systems for optical fiber sensing for determining applicator geometries during interventional therapy in real-time.

Brachytherapy can be used for the treatment of malignant tumors by employing ionizing radiation. One important challenge with brachytherapy can be to ensure that delivery of radiation dose is performed accurately, according to a pre-procedural plan. This challenge includes, for example, the need to accurately position brachytherapy sources, and to compensate for any deviations from the plan as may arise from positioning errors, target volume deformation such as from tissue swelling or changes in radiation transport properties as from the formation of an edema in a surgical cavity. Existing methods generally rely on imaging information, which can provide snapshots of applicator and/or seed positions in time. Implanted beacons have been used to detect organ movement. These approaches can be considered limited in spatial accuracy and/or timeliness of the detection of changes.

US 2009/0137952 A1 discloses robotic medical instrument systems and associated methods utilizing optical fiber sensors. An optical fiber is coupled to an elongate instrument body and includes a fiber core having one or more Bragg gratings. A controller is configured to initiate various actions in response thereto. The controller may generate and display a graphical representation of the instrument body and depict one or more position and/or orientation variables thereof, or adjust motors of an instrument driver to reposition the catheter or another instrument.

The invention is defined in claims 1 and 10. According to the invention, a system for monitoring changes during therapy and a workstation for monitoring changes during therapy according to the independent claims is provided.

In accordance with the present principles, a system for monitoring changes during therapy includes a first probing segment having an optical fiber sensor disposed therein. The first segment is percutaneously inserted in or near a target area and providing a local reference for one or more treatment devices. A second probing segment has an optical fiber sensor disposed therein. The second segment is generally disposed apart from the first probe and provides a spatial reference point for the first segment. The first and second segments have at least one common position to function as a reference between the first and second probes. A shape determination module is configured to determine a shape of each of the first and second segments based on feedback signals to measure changes in the shapes during a procedure and update a therapy plan in accordance with the changes.

A workstation for monitoring changes during therapy includes a computer including a processor and memory. A shape determination module is stored in the memory and configured to determine shapes of a plurality of flexible probes based on feedback signals. The flexible probes each have an optical fiber sensor disposed therein. The flexible probes are configured to measure changes in the shapes during a procedure to provide reference locations for one or more treatment devices. A therapy plan module is stored in the memory and configured to compare and update a therapy plan based upon the reference locations for the one or more treatment devices. The plurality of probes has at least one common position to function as a reference between the probes. The probes include a first probe for being percutaneously inserted in or near a target area, and a second probe for being generally disposed apart from the first probe and providing a spatial reference point for the first probe.

These and other objects, features and advantages of the present disclosure will become apparent from the following detailed description of illustrative embodiments thereof, which is to be read in connection with the accompanying drawings.

This disclosure will present in detail the following description of preferred embodiments with reference to the following figures wherein:
FIG. 1 is a block/flow diagram showing a system and/or workstation for conducting adaptive therapy in accordance with the present principles;
FIG. 2 is a cross-sectional view of a probe showing optical fiber sensors arranged in accordance with one illustrative embodiment;
FIG. 3 is a diagram showing a conceptual arrangement employing multiple optical fiber sensors in accordance with one embodiment; and
FIG. 4 is a flow diagram showing steps for conducting therapy and making real-time adjustments in accordance with an illustrative embodiment.

In accordance with the present principles, systems are provided to determine, estimate and/or visualize (in real time, during therapy), positions and path trajectories of sources for focal energy deposition. Focal energy deposition may include, e.g., radiation sources for brachytherapy or other therapies, cryotherapy probes, applicators for laser ablation, photodynamic therapy, high-intensity focused ultrasound, or other forms of minimally invasive local ablation, etc. The systems may be employed to relate these determinations and/or estimates in real time to a therapy plan and to intra-procedural imaging or other biophysical monitoring, so as to quantify potential deviations from the plan. This results in triggering and guiding suitable adaptations of therapy, so as to realize the therapy goal in spite of deviations from the original therapy plan.

In one embodiment, a collection of (precisely) known source locations within a tissue anatomy of interest can be combined with known characteristics about source-tissue interaction to calculate a final spatial distribution of dose delivery. For example, this information can be related to clinical outcomes on a patient specific basis, which can be used to create a library / database / atlas for, e.g., future optimization of such procedures, physicians-in-training, clinical outcomes studies, and/or patient-specific atlas/database driven automation of seed placement. The present principles may be employed to overcome a wide range of problems and/or disadvantages, including estimating deviations from planned delivery positions in real time throughout the positioning of brachytherapy sources, and for appropriate compensation. Further, the exemplary systems and methods may also be applicable to other minimally or non- invasive therapy modalities that utilize focal energy deposition, such as cryotherapy, RF ablation, laser ablation, photodynamic therapy, high-intensity focused ultrasound, etc.

The present principles will be generically described with respect to an interventional procedure. The interventional procedure may take many forms. A few non-limiting examples will be explained herein for illustrative purposes. In one embodiment, brachytherapy can be used for the treatment of malignant tumors with ionizing radiation. It is generally considered that there are two main forms of brachytherapy. These include permanent seed implantation and high-dose-rate brachytherapy. Permanent seed implantation, which can also be referred to as low-dose-rate brachytherapy, employs rice kernel-sized metallic seeds containing radioactive isotopes such as ¹²⁵I or ¹⁰³Pd. The isotopes are inserted permanently through a needle or catheter into a treatment volume. Usually, needles or catheters are inserted sequentially along different trajectories, and the seeds are delivered into the target tissue at positions along an insertion track of the needle or catheter while the needle or catheter is retracted. The radiation dose can be delivered over a period of weeks to months. High-dose-rate brachytherapy, which involves the placement of multiple catheters within which a radioactive isotope, or a miniaturized x-ray source, can be inserted and moved to defined positions for defined times ("afterloading") so as to deliver internal radiation therapy in several sessions over the course of, e.g., 2 days. After the treatment course, the catheters can be removed. The catheters can form a parallel bundle, or meridian-like lines along the surface of a balloon applicator.

With both forms of brachytherapy, delivery of a correct radiation dose to the tumor is needed, and the radiation dose received by adjacent tissues should be minimized. Planning with pre-procedural images is often used to tailor the dose distribution to a patient's anatomy. Computed tomography (CT), magnetic resonance (MR) or ultrasound (US) images can be utilized to provide relatively high contrast for many tumor types.

With brachytherapy, compensation for positioning errors, catheter displacement, target volume deformation such as from tissue swelling, or changes in radiation transport properties as from the formation of an edema in a surgical cavity needs to be taken into account. For example, if significant deviations in source positions, target tissue geometry, or target volume content occur, planning that was performed with pre-procedural images will likely no longer be relevant and, as a result, the tumor can be inadequately treated and adjacent tissues can be placed at risk for radiation damage.

In accordance with preferred embodiments, optical shape sensing is employed to assist in tracking components for patient treatment. In particularly useful embodiments, a three-dimensional (3D) shape of a flexible, elongated structure can be tracked in real-time. The fundamental principles underlying optical shape sensing, as implemented, for example, with Fiber Bragg Gratings (FBGs) and with Rayleigh scattering are relied upon to provide detailed spatial information for tools during a procedure. With optical shape sensing, the 3D position of a particular location on a patient can be tracked. This sensing method can also allow for tracking of the 3D shape of a deformable mesh that can be tightly fitted around a patient's body, for example.

Relative to other tracking methods such as electromagnetic (EM) tracking, optical shape sensing can have the advantage of immunity to external fields and to distortions of EM fields that can occur due to the presence of metallic objects (e.g., a common occurrence in clinical practice).

It should be understood that the present invention will be described in terms of medical instruments; however, the teachings of the present invention are much broader and are applicable to any instruments employed in tracking or analyzing complex biological or mechanical systems. In particular, the present principles are applicable to internal tracking procedures of biological systems, procedures in all areas of the body such as the lungs, gastrointestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), DVD and Blu-Ray™.

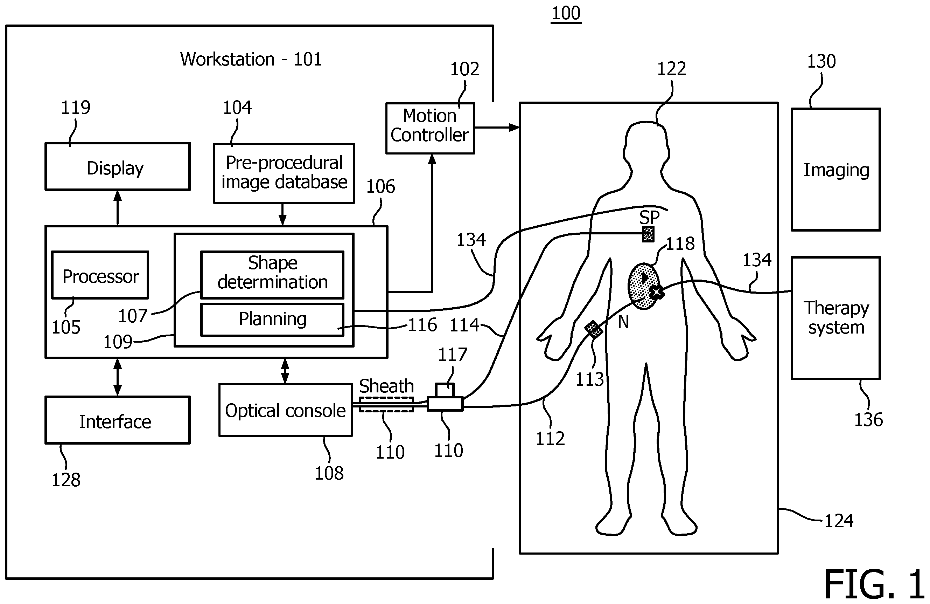

Referring now to the drawings in which like numerals represent the same or similar elements and initially to FIG. 1, a system 100 for focal energy deposition for minimally or non-invasive local tissue ablation is shown in accordance with one illustrative embodiment. System 100 may be part of a therapy planning and monitoring workstation 101 that links shape sensing information, source-tissue interaction modeling, dose monitoring, and clinical outcomes databasing on a patient-specific basis for procedure optimization, reporting, and physician training. System 100 may include an image database or memory storage 104. The database 104 stores images, preferably three-dimensional (3D) images, of a patient 122 on which a procedure is to be performed. System 100 includes a computer 106 having a processor 105 capable of executing a shape sensing determination algorithm or method 107 stored in memory 109.

An optical console 108 is controlled by and provides data to the computer 106. The optical console 108 delivers light to optical fiber probes 112 and 114 and receives light from the probes 112 and 114. The probes 112 and 114 are flexible, and each encloses an optical fiber shape sensor or sensors. It should be noted that the number of probes may be greater than two or a single probe may be employed. The first probe 112 and second probes 114 may include two segments on separate tethers or may include different sub-sections or segments on a same tether. It should also be understood that each probe 112, 114 may include a plurality of optical fibers, e.g., 2, 3, 4 or more fibers. Optical fiber shape sensing may take many forms and may employ different technologies, such as, e.g., multi core geometry, interferometer, polarization diversity, laser characteristics, etc. Shape sensing is illustratively described in, e.g., Published Patent Application US2011/0109898, to Froggatt et al.

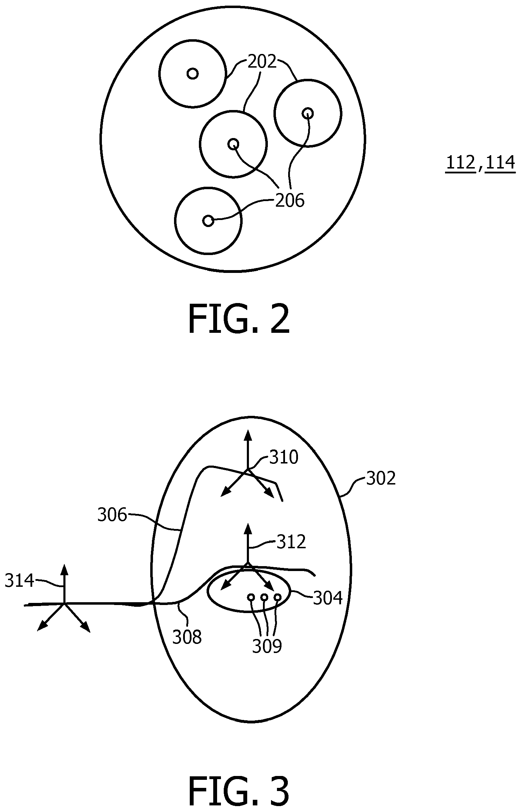

Referring to FIG. 2 with continued reference to FIG. 1, an axial cross section of the optical fiber probe 112, 114 is shown with optical sensors 202. Sensors 202 include four fibers with single mode cores 206 constrained within an elongated body with a circular cross section. Light from the optical console 108 can be directed to each fiber, either simultaneously or sequentially. In each fiber 202, light is reflected at different distances, either with FBGs or with Rayleigh scattering, depending on how the optical fiber 202 is constructed. Preferably, the optical fibers 202 are arranged in a substantially helical fashion along the length of the sensor. This is one exemplary design. Other exemplary designs or technologies in accordance with the present principles may be employed.

Referring again to FIG. 1, the computer 106 may include memory 109 that stores a shape determination algorithm or method 107 for determining the shapes and positions of the flexible probes 112 and 114 based on measurements obtained from the optical console 108. In one exemplary embodiment, at least one of the probes 112 can be inserted into a body of the patient 122 percutaneously, to a location in or very close to a region of tissue 118 that will be targeted by focal energy deposition. A feedback loop can be provided between the shape determination algorithm 107 and a therapy system 136 or planning module 116 such that the region of tissue 118 targeted (by the therapy system 136 and/or planning module 116) and the intensity of treatment can be altered based on the output or updates of the shape determination algorithm 107, e.g., due to movement, swelling, edema, etc.

Preferably, there are one or more reference points along a single probe or several such probes for referencing that can characterize a localization/orientation of the patient anatomy near the treatment volume (118). The location of the first flexible probe 112 can therefore be tracked relative to the location of the reference points (due to e.g., probe 114). In the case that a second flexible probe 114 is employed as a reference, the second flexible probe 114 may be mechanically attached or coupled to the first flexible probe 112 on a proximal end, to ensure that errors in the output of the shape determination algorithm 107 are as similar as possible for both probes. The mechanical attachment between the two probes may also be realized with coupling of the second probe 114 to first probe 112 at a mid-segment position along probe 112 so long as the attachment point and associated tracking errors at that point are characterized and accounted for when tracking the second probe 114. The coupling of the probes 112 and 114 may be provided using a sheath 110 or other device to couple the proximal ends of the probes 112 and 114 together. For the case where the segments (probes) are on a same tether (in-line), a common reference point would be a shared connection point between the two segments.

There can be additional markers on the probes 112, 114 at the one or more reference locations along the lengths of the probes 112, 114 (e.g., multiple sheaths 110 or connecting points with, e.g., EM markers 117) that can be tracked with methods other than optical shape sensing. The information from these markers 117 can be incorporated into the shape determination algorithm 107 in real time to improve the accuracy of the shape determination algorithm 107. These exemplary markers 117 may not provide information about the spatial locations of the probe tips themselves but can provide information about the spatial locations of more proximal parts of the probes 112, 114 that could nonetheless present boundary conditions that are useful for the shape determination algorithm 107.

In one embodiment, the patient 122 may be positioned on a table 124 that can be translated with a motion controller 102. The tumor region 118 is an intended radiotherapy target. The system 100 may include a device 113 for delivering radiation, which may include a needle, catheter or other device 113 for implanting seeds or focusing radiation in the tumor 118. The device 113 is connected to the probe 112 so that the locations of therapeutic devices (e.g., radiation seeds) and their placement trajectories are known using the optical fiber sensing feedback from the probe 112. Other radiation delivery or therapy systems 136 may also be employed. One flexible probe 112 (which includes an optical fiber sensor or sensors) extends from the optical console 108 and joins a therapy application catheter (N) 113, and follows the catheter 113 to the tumor region 118.

The second flexible probe 114 which includes another optical fiber sensor is extended from the optical console 108 to a fixed region or surface point (SP) on the surface of the patient 122. In this instance, close to the optical console 108, the two optical fiber sensors of probes 112 and 114 are enclosed within the sheath 110 that is designed to permit minimal torsion, with bending radii that are as large as possible without compromising functionality. Inside the sheath 110, the two optical fiber sensors are either kept at fixed angulations with respect to each other or if they are freely moving relative to one another, the location and orientation of the fibers are tracked or otherwise determined relative to one another and relative to a common reference in a laboratory or environmental coordinate system. The optical console 108 interrogates or polls for measurements from each of the two fiber sensors either serially in a temporally-defined (or time-stamped) rapid interleaved fashion or in parallel with simultaneous readings from both fiber sensors (depending on the configuration of the interrogation platform). This concept can be extended further to more than 2 fibers to enable guidance to multiple treatment sites in parallel.

The data from the optical console 108 can be received by the computer 106, which processes the data with the shape determination algorithm 107. In this way, the computer 106 derives estimates of the 3D shapes of the optical fibers in probes 112 and 114. The shape determination algorithm 107 may include as a boundary condition the fact that the two optical fiber sensors are mechanically constrained or of known position / orientation relative to one another within the sheath 110. The relative position/orientation of the two fiber sensor reference locations can alternately be derived from actual measurements of reference markers and external tracking with a secondary position/orientation measurement device (e.g., 117).

After deriving these determinations and/or estimates, the computer 106 can compare them with determinations, therapy plans and/or estimates from previous time points to determine whether applicator movement has occurred relative to the body of the patient 122, as referenced by the second flexible probe 114.

In one embodiment, the therapy plan module/system 116 may be stored or have a portion stored in memory 109. The therapy plan module/system 116 may be configured to compare planned placements of the one or treatment devices (e.g., seeds, catheters, etc.) with actual placements of the one or treatment devices based on path trajectories determined from the probe 112. The therapy plan module 116 is configured to output subsequent locations for placement of the one or treatment devices based upon achieving overall plan objectives. The therapy plan module 116 may include weighting systems with alternative roadmaps and / or clinical decision trees based on prior history so that evaluations of current feedback can be employed to ensure that the objectives of the treatment plan are met.

If there is no relative movement between the actual measurements and the therapy plan, the 3D shapes of the parts of the sensors that are fixed to the patient 122 (probes 112 and 114) could be expected to move synchronously (i.e., together). Otherwise, they would have asynchronous movement indicating a change. The impact of any detected relative motion is then determined with knowledge of the patient's anatomy obtained with the pre-procedural image database 104 and with information derived from similar prior studies in a database or library linking interventional path characteristics with clinical outcomes. If the relative movement exceeds defined alarm limits, a warning is given and therapy is suspended pending an adaption of the therapy plan according to the changed applicator geometry. The warning may be produced audibly, visually on a display 119 or by any other suitable method including haptic feedback within the probe itself.

Programming, device control, monitoring of functions and/or any other interactions with the computer 106 or workstation 101 may be performed using an interface 128. Display 119 may also permit a user to interact with the workstation 101 and its components and functions, or any other element within the system 100. This is further facilitated by the interface 128 which may include a keyboard, mouse, a joystick, a haptic device, or any other peripheral or control to permit user feedback from and interaction with the workstation 101 or system 100.

In another exemplary embodiment, the flexible probe 112 with the optical fiber sensor can be inserted to reach the tumor region with endovascular access. For example, the probe 112 can be advanced through the patient's vasculature until it is close to the tumor region 118. The probe 112 is then inserted through the vascular wall towards the tumor region 118. This exemplary procedure can likely be performed with fluoroscopic guidance using an imaging system 130 in combination with radio opaque contrast agents, for example. Other imaging techniques may also be employed. More than one flexible probe (112) can be inserted into the tumor region 118. More than one flexible probe (114) can also be placed at an additional point outside the tumor region 118 to monitor a position of the patient 122 or other equipment employed during the procedure.

In one illustrative embodiment, flexible probes 134 (e.g., therapy applicators or other devices) can be tracked and/or imaged independently of the optical console 108. For example, data from these tracking/imaging methods can be used by the shape determination algorithm/procedure 107 to impose additional geometric constraints on the spatial positions of the probes 112, 114, to, e.g., improve the accuracy of the 3D shape estimates and/or delineate physiological structures (both target and normal tissue) in the volume of interest (118). In this way, independent reference points are created using tracking technologies such as EM, imaging (imaging system 130), etc., which permit a check on the position/orientations of the other probes 112, 114 employed in the procedure. Imaging device 130 and therapy system 136 may be connected to the workstation 101. In this way, the imaging system 130 and the therapy system 136 can be controlled by and/or provide feedback to the rest of the system 100.

In another embodiment, a greater number of flexible probes equipped with fiber optic shape sensors may be employed to monitor flexible surfaces and/or volumes. These surfaces or volumes may or may not be a target of interest such as a tumor, but instead may include patient movements, movements of internal organs, movement of a table 124 or other platform, movements of instruments etc. A region may include one or more optical fiber shape sensors that can be used to derive estimates of 2D or 3D changes in applicator and/or tissue geometry that can occur inside and/or outside the tumor region 118. Such an embodiment can be more complex and can likely utilize more than two optical fiber shape sensors (probes 112 or 114). This can likely provide more information about the movement of the therapy applicators relative to target and/or normal tissues, for example. The system 100 can be made modular to easily accommodate a plurality of flexible probes with fiber optic sensors.

Referring to FIG. 3, a diagram shows an illustrative conceptual embodiment in accordance with the present principles. A mass 302 capable of movement includes a flexible material target 304. Both the mass 302 and the target 304 may vibrate or change position in three dimensions. The mass 302 and the target 304 may move a limited amount relative to each other but may also move together. It is necessary to understand the relative movement and the global movement of both the mass 302 and the target 304 to account for the dynamic behavior of both. In accordance with the present principles, optical fiber sensors 306, 308 are employed to locally monitor the positional/orientation changes of the mass 302 and the target 304. The fiber optical sensor 308 can detect local strains relative to a local coordinate system 312 with a high resolution. Therefore, an optical fiber sensor 308 is placed at or near the target 304 to detect local strains.

Similarly, local strain (e.g., positions / movement) of the mass 302 needs to be monitored relative to its local coordinate system 310. In this case, a fiber optic sensor 306 may be placed on the mass 302 to monitor local changes. In addition, positions of the target 304, mass 302 and devices 309 (e.g., radiation sources, seeds, etc.) need to be understood in accordance with a more global coordinate system 314. The devices 309 are positioned by a tracked tool and so their positions are accurately known. Additional information relating the coordinates systems 310 and 312 can be provided by various constraints and boundary conditions. For example, the optical fiber sensors 306 and 308 may share a common position or positions, or are otherwise tracked so that the reference point on one fiber is known relative to the reference point on the other. As described above, this may be achieved by bundling portions of the optical fibers for both sensors 306 and 308 (e.g., in a sheath or the like). In this way, a common reference or constraint is known and a solution to all other points along the optical fiber sensors 306 and 308 can be determined and monitored relative to a common coordinate system 314.

In the case described above for radiation therapy, the monitoring of the target 304 may include changes due to swelling of target areas, due to patient activity, due to movement of equipment, due to error in placement of device 309, etc. Having another reference point or points from sensors 306 provides not only local movements for another position on the patient but also knowledge of the orientation of the mass 302 (e.g., patient on a table or platform, etc.) relative to the other sensor 308. In the example, this can provide guidance for aiming / focusing radiation, placement of radiation seeds, etc.

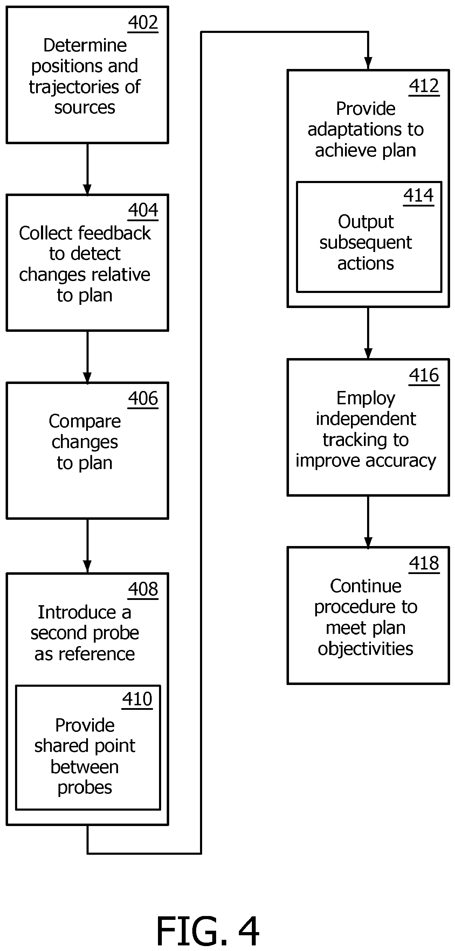

Referring to FIG. 4, a method for monitoring changes during therapy is illustratively shown. In block 402, positions and path trajectories of sources for focal energy deposition are determined by introducing at least one flexible probe percutaneously into a body to a location in or close to a region of tissue targeted for the focal energy deposition. The probe includes at least one optical fiber sensor. In one embodiment, the probe may be included with the device (e.g., a needle), which is depositing or placing the sources so that the positions and path trajectories of the sources are easily identified.

In block 404, feedback signals are collected from the at least one flexible probe and input to a shape determination module to detect changes in the at least one flexible probe such that, in accordance with a therapy plan, the region of tissue targeted for therapy and an intensity of treatment can be altered based on the output of the shape determination module.

In block 406, the positions and path trajectories are compared in real time to a therapy plan to quantify deviations from the plan. In block 408, a second probe may be introduced to provide a reference position for the first probe. The second probe also preferably includes at least one optical fiber sensor. The at least one flexible probe and the second probe may share at least one common position to provide a constraint or condition for correlating coordinate systems in block 410, or each probe may have reference positions that are known relative to one another.

In block 412, adaptations of therapy are provided to achieve a therapy goal based on the collected feedback and plan comparisons. The deviations are accounted for in subsequent activities in the procedure. In block 414, subsequent locations for placement of the one or treatment devices based upon achieving overall plan objectives may be output. Other changes, suggestions or actions may be called for to ensure that the therapy plan goals are achieved. In block 416, an additional tracking device or devices configured to independently track paths of at least one flexible probe may be employed to verify and/or improve position measurements of the at least one flexible probe or otherwise provide solution constraints or conditions for determining positional information for sensor probes. In block 418, the procedure continues until completed, e.g., the plan objectives are met.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for systems and methods for optical fiber sensing for determining real time changes in applicator geometry for interventional therapy (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. A system for monitoring changes during therapy, comprising:
a first segment (112) having an optical fiber sensor disposed therein, the first segment for being percutaneously inserted in or near a target area and providing a local reference for one or more treatment devices;
a second segment (114) having an optical fiber sensor disposed therein, the second segment being generally disposed apart from the first segment and providing a spatial reference point for the first segment, and the first and second segments having at least one common position to function as a reference between the first and second segments, **characterized by**
a shape determination module (107) configured to determine, by using the at least one common position of the first and second segments (112, 114) as a boundary condition, a shape of each of the first and second segments based on feedback signals to measure changes in the shapes during a procedure by comparing the shapes with shape information from a different point in time, and to relate the measured changes in the shapes to a therapy plan so as to quantify deviations from the therapy plan and update the therapy plan in accordance with the changes.

2. The system as recited in claim 1, wherein the shape determination module (107) receives optical signals to determine path trajectories of the first segment and positioning of the one or more treatment devices is determined based upon the path trajectories.

3. The system as recited in claim 2, wherein the one or more treatment devices include medical instruments carrying radiation sources (309).

4. The system as recited in claim 2, wherein the one or more treatment devices include radiation seeds (309) and the path trajectories are employed to determine seed locations.

5. The system as recited in claim 2, wherein the one or more treatment devices include focal treatment radiation sources.

6. The system as recited in claim 1, further comprising a therapy plan module (116) configured to compare planned placements of the one or treatment devices with actual placements of the one or treatment devices based on the path trajectories, the therapy plan module being configured to output subsequent locations for placement of the one or treatment devices based upon achieving overall plan objectives.

7. The system as recited in claim 1, further comprising an additional tracking device (134) configured to independently track paths of at least one of the first segment and the second segment to verify positions of the at least one of the first segment and the second segment.

8. The system as recited in claim 1, wherein the at least one common position includes a tracking device (117) to track a position of the at least one common position.

9. The system as recited in claim 1, wherein the first and second segments include two separate tethers or include different sub-sections of a same tether.

10. A workstation for monitoring changes during therapy, comprising:
a computer (106) including a processor (105) and memory (109);
a shape determination module (107) stored in the memory and configured to determine shapes of a plurality of flexible probes (112, 114) based on feedback signals, the probes (112, 114) having at least one common position (110) to function as a reference between the probes and including a first probe (112) for being percutaneously inserted in or near a target area and a second probe (114) for being generally disposed apart from the first probe and providing a spatial reference point for the first probe, wherein the shape determination module (107) is configured to determine the shapes of the probes (112, 114) by using the at least one common position (110) of the first and second probes (112, 114) as a boundary condition, the flexible probes each having an optical fiber sensor disposed therein, **characterized in that** the flexible probes being configured to measure changes in the shapes during a procedure by comparing the shapes with information from a different point in time to provide reference locations for one or more treatment devices; and
a therapy plan module (116) stored in the memory and configured to relate the measured changes in the shapes of the probes (112, 114) to a therapy plan based upon the reference locations for the one or more treatment devices and update the therapy plan.

11. The workstation as recited in claim 10, wherein the shape determination module (107) receives optical signals to determine path trajectories of the first probe and positioning of the one or more treatment devices is determined based upon the path trajectories.

12. The workstation as recited in claim 10, wherein the one or more treatment devices include radiation seeds (309) and the path trajectories are employed to determine seed locations.

13. The workstation as recited in claim 10, wherein the therapy plan module (116) is configured to compare planned placements of the one or treatment devices with actual placements of the one or treatment devices based on the path trajectories, the therapy plan module being configured to output subsequent locations for placement of the one or treatment devices based upon achieving overall plan objectives.

14. The workstation as recited in claim 10, further comprising an additional tracking device (134) configured to independently track paths of at least one of the first probe and the second probe to verify positions of the at least one of the first probe and the second probe.

15. The workstation as recited in claim 10, wherein the at least one common position (110) includes a tracking device (117) to track a position of the at least one common position.

## Patentansprüche

1. System zur Überwachung von Veränderungen während einer Therapie, umfassend:
ein erstes Segment (112) mit einem darin angeordneten faseroptischen Sensor, wobei das erste Segment zum perkutanen Einführen in oder in der Nähe eines Zielbereichs sowie zum Bereitstellen einer lokalen Referenz für ein oder mehrere Behandlungsvorrichtungen vorgesehen ist;
ein zweites Segment (114) mit einem darin angeordneten faseroptischen Sensor, wobei das zweite Segment im Allgemeinen von dem ersten Segment beabstandet angeordnet ist und einen räumlichen Referenzpunkt für das erste Segment vorsieht, und wobei das erste und zweite Segment mindestens eine gemeinsame Position aufweisen, um als eine Referenz zwischen dem ersten und zweiten Segment zu wirken,
**gekennzeichnet durch**
ein Formermittlungsmodul (107), das so konfiguriert ist, dass es, unter Verwendung der mindestens einen gemeinsamen Position des ersten und zweiten Segments (112, 114) als eine Grenzbedingung, eine Form jedes, des ersten und zweiten, Segments aufgrund von Rückführsignalen ermittelt, um während einer Prozedur durch Vergleichen der Formen mit Forminformationen von einem anderen Zeitpunkt Veränderungen in den Formen zu messen und die gemessenen Veränderungen in den Formen mit einem Therapieplan in Zusammenhang zu bringen, um Abweichungen von dem Therapieplan zu quantifizieren und den Therapieplan den Veränderungen entsprechend zu aktualisieren.

2. System nach Anspruch 1, wobei das Formermittlungsmodul (107) optische Signale zum Ermitteln von Bahntrajektorien des ersten Segments empfängt und das Positionieren der einen oder mehrerer Behandlungsvorrichtungen aufgrund der Bahntrajektorien ermittelt wird.

3. System nach Anspruch 2, wobei die eine oder mehrere Behandlungsvorrichtungen Strahlungsquellen (309) tragende, medizinische Instrumente enthalten.

4. System nach Anspruch 2, wobei die eine oder mehrere Behandlungsvorrichtungen Strahlungssaaten (309) enthalten und die Bahntrajektorien zum Ermitteln von Saatpositionen eingesetzt werden.

5. System nach Anspruch 2, wobei die eine oder mehrere Behandlungsvorrichtungen Strahlungsquellen zur fokalen Behandlung enthalten.

6. System nach Anspruch 1, weiterhin umfassend ein Therapieplanmodul (116), das so konfiguriert ist, dass es geplante Platzierungen der einen oder mehrerer Behandlungsvorrichtungen mit aktuellen Platzierungen der einen oder mehrerer Behandlungsvorrichtungen aufgrund der Bahntrajektorien vergleicht, wobei das Therapieplanmodul so konfiguriert ist, dass es nachfolgende Positionen zur Platzierung der einen oder mehrerer Behandlungsvorrichtungen auf der Grundlage des Erreichens von Gesamtplanzielen ausgibt.

7. System nach Anspruch 1, weiterhin umfassend eine zusätzliche Tracking-Vorrichtung (134), die so konfiguriert ist, dass sie Wege von zumindest dem ersten Segment oder dem zweiten Segment unabhängig verfolgt, um Positionen von zumindest dem ersten Segment oder dem zweiten Segment zu verifizieren..

8. System nach Anspruch 1, wobei die mindestens eine gemeinsame Position eine Tracking-Vorrichtung (117) enthält, um eine Position der mindestens einen gemeinsamen Position zu verfolgen.

9. System nach Anspruch 1, wobei das erste und zweite Segment zwei separate Haltelemente enthalten oder verschiedene Subabschnitte eines gleichen Halteelements enthalten.

10. Workstation zur Überwachung von Veränderungen während einer Therapie, umfassend:
einen Computer (106) mit einem Prozessor (105) und Speicher (109);
ein Formermittlungsmodul (107), das in dem Speicher gespeichert und so konfiguriert ist, dass es Formen einer Mehrzahl flexibler Sonden (112, 114) aufgrund von Rückführsignalen ermittelt, wobei die Sonden (112, 114) mindestens eine gemeinsame Position (110) aufweisen, um als eine Referenz zwischen den Sonden zu wirken, und eine erste Sonde (112), um in oder in die Nähe eines Zielbereichs perkutan eingeführt zu werden, sowie eine zweite Sonde (114), um von der ersten Sonde generell beabstandet angeordnet zu sein und einen räumlichen Referenzpunkt für die erste Sonde vorzusehen, enthalten, wobei das Formermittlungsmodul (107) so konfiguriert ist, dass es die Formen der Sonden (112, 114) unter Verwenden der mindestens einen gemeinsamen Position (110) der ersten und zweiten Sonde (112, 114) als eine Grenzbedingung ermittelt, wobei die flexiblen Sonden jeweils einen darin angeordneten faseroptischen Sensor aufweisen,
**dadurch gekennzeichnet, dass**
die flexiblen Sonden so konfiguriert sind, dass sie Veränderungen in den Formen während einer Prozedur durch Vergleichen der Formen mit Informationen von einem anderen Zeitpunkt messen, um Referenzpositionen für eine oder mehrere Behandlungsvorrichtungen vorzusehen; sowie
ein Therapieplanmodul (116), das in dem Speicher gespeichert und so konfiguriert ist, dass es die gemessenen Veränderungen in den Formen der Sonden (112, 114) aufgrund der Referenzpositionen für die eine oder mehrere Behandlungsvorrichtungen in Beziehung zu einem Therapieplan setzt und den Therapieplan aktualisiert.

11. Workstation nach Anspruch 10, wobei das Formermittlungsmodul (107) optische Signale empfängt, um Bahntrajektorien der ersten Sonde zu ermitteln und das Positionieren der einen oder mehrerer Behandlungsvorrichtungen aufgrund der Bahntrajektorien ermittelt wird.

12. Workstation nach Anspruch 10, wobei die eine oder mehrere Behandlungsvorrichtungen Strahlungssaaten (309) enthalten und die Bahntrajektorien zum Ermitteln von Saatpositionen eingesetzt werden.

13. Workstation nach Anspruch 10, wobei das Therapieplanmodul (116) so konfiguriert ist, dass es geplante Platzierungen der einen oder mehrerer Behandlungsvorrichtungen mit aktuellen Platzierungen der einen oder mehrerer Behandlungsvorrichtungen aufgrund der Bahntrajektorien vergleicht, wobei das Therapieplanmodul so konfiguriert ist, dass es nachfolgende Positionen zur Platzierung der einen oder mehrerer Behandlungsvorrichtungen auf der Grundlage des Erreichens von Gesamtplanzielen ausgibt.

14. Workstation nach Anspruch 10, weiterhin umfassend eine zusätzliche Tracking-Vorrichtung (134), die so konfiguriert ist, dass sie Wege von zumindest der ersten Sonde oder der zweiten Sonde unabhängig verfolgt, um Positionen von zumindest der ersten Sonde oder der zweiten Sonde zu verifizieren.

15. Workstation nach Anspruch 10, wobei die mindestens eine gemeinsame Position (110) eine Tracking-Vorrichtung (117) enthält, um eine Position der mindestens einen gemeinsamen Position zu verfolgen.

## Revendications

1. Système de surveillance des changements pendant un traitement, comprenant :
un premier segment (112) ayant un capteur à fibre optique disposé à l'intérieur, le premier segment étant destiné à être inséré par voie percutanée dans ou près d'une zone cible et fournissant un repère local pour un ou plusieurs dispositifs de traitement ;
un second segment (114) ayant un capteur à fibre optique disposé à l'intérieur, le second segment étant généralement disposé indépendant du premier segment et fournissant un repère dans l'espace pour le premier segment, et le premier et le second segments ayant au moins une position commune pour fonctionner comme un repère entre le premier et le second segments, **caractérisé par**
un module de détermination de forme (107) configuré pour déterminer, en utilisant ladite au moins une position commune du premier et du second segments (112, 114) comme condition limite, une forme de chacun des premier et second segments basée sur des signaux de retour pour mesurer des changements de formes pendant une procédure, en comparant les formes avec des informations de forme d'un point différent dans le temps, et pour associer les changements mesurés dans les formes à un plan de traitement de façon à quantifier des écarts par rapport au plan de traitement et à mettre à jour le plan de traitement conformément aux changements.

2. Système selon la revendication 1, dans lequel le module de détermination de forme (107) reçoit des signaux optiques pour déterminer des trajectoires de chemin du premier segment et le positionnement du ou des dispositifs de traitement est déterminé sur la base des trajectoires de chemin.

3. Système selon la revendication 2, dans lequel le ou les dispositifs de traitement incluent des instruments médicaux porteurs de sources de rayonnement (309).

4. Système selon la revendication 2, dans lequel le ou les dispositifs de traitement incluent des grains de rayonnement (309) et les trajectoires de chemin sont utilisées pour déterminer les emplacements de grains.

5. Système selon la revendication 2, dans lequel le ou les dispositifs de traitement incluent des sources de rayonnement de traitement focal.

6. Système selon la revendication 1, comprenant en outre un module de plan de traitement (116) configuré pour comparer des placements planifiés du ou des dispositifs de traitement aux placements effectifs du ou des dispositifs de traitement, sur la base des trajectoires de chemin, le module de plan de traitement étant configuré pour émettre des emplacements successifs pour le placement du ou des dispositifs sur la base de la réalisation d'objectifs globaux du plan.

7. Système selon la revendication 1, comprenant en outre un dispositif de suivi supplémentaire (134) configuré pour suivre indépendamment des chemins d'au moins un parmi le premier segment et le second segment pour vérifier des positions dudit au moins un premier segment et un second segment.

8. Système selon la revendication 1, dans lequel ladite au moins une position commune inclut un dispositif de suivi (117) pour suivre une position de ladite au moins une position commune.

9. Système selon la revendication 1, dans lequel le premier et le second segments incluent deux attaches séparées ou incluent différentes sous-sections d'une même attache.

10. Poste de travail pour surveiller des changements pendant un traitement, comprenant :
un ordinateur (106) incluant un processeur (105) et une mémoire (109) ;
un module de détermination de forme (107) stocké dans la mémoire et configuré pour déterminer des formes d'une pluralité de sondes flexibles (112, 114), sur la base de signaux de retour, les sondes (112, 114) ayant au moins une position commune (110) pour fonctionner comme un repère entre les sondes et incluant une première sonde (112) à insérer par voie percutanée dans ou près d'une zone cible et une seconde sonde (114) à disposer indépendamment de la première sonde et fournissant un point de repère dans l'espace pour la première sonde, dans lequel le module de détermination de forme (107) est configuré pour déterminer les formes des sondes (112, 114) en utilisant ladite au moins une position commune (110) de la première et de la seconde sondes (112, 114) comme condition limite, les sondes flexibles ayant chacune un capteur à fibre optique disposé à l'intérieur,
**caractérisé en ce que**
les sondes flexibles étant configurées pour mesurer des changements de formes pendant une procédure en comparant les formes à des informations provenant d'un point différent dans le temps pour fournir des emplacements de référence pour un ou plusieurs dispositifs de traitement ; et
un module de plan de traitement (116) stocké dans la mémoire et configuré pour associer les changements mesurés des formes de sondes (112, 114) à un plan de traitement sur la base des emplacements de référence pour le ou les dispositifs de traitement et mettre à jour le plan de traitement.

11. Poste de travail selon la revendication 10, dans lequel le module de détermination de forme (107) reçoit des signaux optiques afin de déterminer des trajectoires de chemin de la première sonde et le positionnement du ou des dispositifs de traitement est déterminé sur la base des trajectoires de chemin.

12. Poste de travail selon la revendication 10, dans lequel le ou les dispositifs de traitement incluent des grains de radiation (309) et les trajectoires de chemin sont utilisées pour déterminer des emplacements de grains.

13. Poste de travail selon la revendication 10, dans lequel le plan de module de traitement (116) est configuré pour comparer des emplacements planifiés du ou des dispositifs de traitement à des emplacements effectifs du ou des dispositifs de traitement sur la base des trajectoires de chemin, le module de plan de traitement étant configuré pour émettre des emplacements successifs pour le placement du ou des dispositifs de traitement sur la base de la réalisation d'objectifs de plan globaux.

14. Poste de travail selon la revendication 10, comprenant en outre un dispositif de suivi supplémentaire (134) configuré pour suivre indépendamment des chemins d'au moins une de la première sonde et de la seconde sonde pour vérifier des positions de ladite au moins première sonde et de la seconde sonde.

15. Poste de travail selon la revendication 10, dans lequel ladite au moins une position commune (110) inclut un dispositif de suivi (117) pour suivre une position de ladite au moins une position commune.
